(19) Europäisches Patentamt
European Patent Office
Office européen des brevets

(11) Veröffentlichungsnummer: **0 185 983**
**A1**

(12) # EUROPÄISCHE PATENTANMELDUNG

(21) Anmeldenummer: 85115352.8

(22) Anmeldetag: 03.12.85

(51) Int. Cl.[4]: C 07 D 271/06
A 01 N 47/28

(30) Priorität: 12.12.84 DE 3445205

(43) Veröffentlichungstag der Anmeldung: 02.07.86 Patentblatt 86/27

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Heinemann, Ulrich, Dr.
Feldstrasse 18
D-5653 Leichlingen 1(DE)

(72) Erfinder: Knops, Hans-Joachim, Dr.
Köpenickerstrasse 35
D-4019 Monheim(DE)

(72) Erfinder: Steinbeck, Karl, Dr.
Rosenkranz 36
D-5093 Burscheid(DE)

(72) Erfinder: Brandes, Wilhelm, Dr.
Eichendorffstrasse 3
D-5653 Leichlingen(DE)

(72) Erfinder: Hänssler, Gerd, Dr.
Am Arenzberg 58a
D-5090 Leverkusen 3(DE)

(72) Erfinder: Reinecke, Paul, Dr.
Steinstrasse 8
D-5090 Leverkusen 3(DE)

(54) **N-(3-Chlor-1,2,4-oxadiazol-5-yl)-harnstoffe.**

(57) N-(3-Chlor-1,2,4-oxadiazol-5-yl)-harnstoffe der allgemeinen Formel (I),

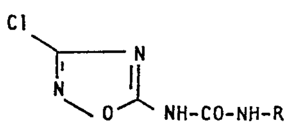

(I)

in welcher
R für Alkyl, Halogenalkyl, für jeweils gegebenenfalls substituiertes Cycloalkyl oder Cycloalkylalkyl oder für gegebenenfalls substituiertes Aryl steht, sowie ihre Verwendung als Schädlingsbekämpfungsmittel.

Die neuen N-(3-Chlor-1,2,4-oxadiazol-5-yl)-harnstoffe können hergestellt werden, indem man 5-Amino-3-chlor-1,2,4-oxadiazol mit geeigneten Isocyanaten umsetzt.

EP 0 185 983 A1

Croydon Printing Company Ltd

BAYER AKTIENGESELLSCHAFT
Konzernverwaltung RP
Patentabteilung

5090 Leverkusen, Bayerwerk

Bas/Kü-c 11.12.84
Ia

N-(3-Chlor-1,2,4-oxadiazol-5-yl)-harnstoffe

Die Erfindung betrifft neue N-(3-Chlor-1,2,4-oxadiazol-5-yl)-harnstoffe, ein Verfahren zu ihrer Herstellung und ihre Verwendung als Schädlingsbekämpfungsmittel, vor allem als Fungizide.

Es ist bereits bekannt, daß Carbamate oder Imide, wie beispielsweise Zink-ethylen-1,2-bis-(dithiocarbamat) oder N-Trichlormethylmercapto-phthalimid, fungizide Eigenschaften besitzen (vgl. z.B. K.H. Büchel "Pflanzenschutz und Schädlingsbekämpfung" Thieme Verlag Stuttgart 1977, S. 137, 138, 140).

Die Wirksamkeit dieser vorbekannten Verbindungen ist jedoch, insbesondere bei niedrigen Aufwandmengen und -konzentrationen, nicht immer in allen Anwendungsbereichen völlig zufriedendstellend.

Es wurden neue N-(3-Chlor-1,2,4-oxadiazol-5-yl)-harnstoffe der allgemeinen Formel (I),

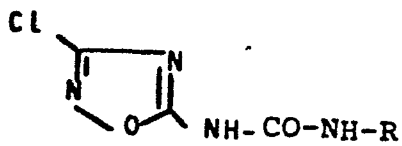

(I)

in welcher

R für Alkyl, Halogenalkyl, für jeweils gegebenenfalls substituiertes Cycloalkyl oder Cycloalkylalkyl oder für gegebenenfalls substituiertes Aryl steht,

gefunden.

Weiterhin wurde gefunden, daß man die neuen N-(3-Chlor-1,2,4-oxadiazol-5-yl)-harnstoffe der allgemeinen Formel (I),

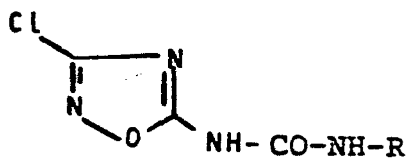

(I)

in welcher

R für Alkyl, Halogenalkyl, für jeweils gegebenenfalls substituiertes Cycloalkyl oder Cycloalkylalkyl oder für gegebenenfalls substituiertes Aryl steht,

erhält, wenn man 5-Amino-3-chlor-1,2,4-oxadiazol der Formel (II),

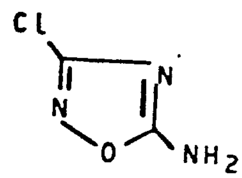

(II)

mit Isocyanaten der Formel (III),

R-N=C=O (III)

in welcher

R die oben angegebene Bedeutung hat,

gegebenenfalls in Gegenwart eines Verdünnungsmittels sowie gegebenenfalls in Gegenwart eines Katalysators umsetzt.

Schließlich wurde gefunden, daß die neuen N-(3-Chlor-1,2,4-oxadiazol-5-yl)-harnstoffe der allgemeinen Formel (I) fungizide Eigenschaften besitzen.

Überraschenderweise zeigen die erfindungsgemäßen N-(3-Chlor-1,2,4-oxadiazol-5-yl)-harnstoffe der allgemeinen Formel (I) eine erheblich bessere Wirksamkeit gegen Schädlinge, insbesondere gegen Pilze, als die aus dem Stand der Technik bekannten Carbamate oder Imide, wie beispielsweise das Zink-ethylen-1,2-bis-(dithiocarbamat) oder das N-Trichlormethylthio-phthalimid, welches wirkungsmäßig naheliegende Verbindungen sind.

Die erfindungsgemäßen N-(3-Chlor-1,2,4-oxadiazol-5-yl)-harnstoffe sind durch die Formel (I) allgemein definiert. Bevorzugt sind Verbindungen der Formel (I), bei welchen

R für jeweils geradkettiges oder verzweigtes Alkyl oder Halogenalkyl mit jeweils 1 bis 12 Kohlenstoffatomen und gegebenenfalls 1 bis 15 gleichen oder verschiedenen Halogenatomen, für jeweils gegebenenfalls einfach oder mehrfach, gleich oder verschieden im Cyloalkylteil durch Halogen und/oder geradkettiges oder verzweigtes Alkyl mit 1 bis 4 Kohlenstoffatomen substituiertes Cycloalkyl oder Cycloalkylalkyl mit jeweils 3 bis 7 Kohlenstoffatomen im Cycloalkylteil und gegebenenfalls 1 bis 6 Kohlenstoffatomen im Alkylteil oder für gegebenenfalls einfach oder mehrfach, gleich oder verschieden substituiertes Aryl mit 6 bis 10 Kohlenstoffatomen steht, wobei als Arylsubstituenten in Frage kommen: Halogen, Cyano, Nitro, sowie jeweils geradkettiges oder verzweigtes Alkyl, Alkoxy, Halogenalkyl, Alkoxyalkyl, Alkoxyalkyloxycarbonyl oder Alkoxycarbonyl mit jeweils 1 bis 8 Kohlenstoffatomen in den einzelnen Alkyl- bzw. Alkoxyteilen und gegebenenfalls 1 bis 9 gleichen oder verschiedenen Halogenatomen.

Besonders bevorzugt sind erfindungsgemäße Verbindungen der Formel (I), bei welchen

R für jeweils geradkettiges oder verzweigtes Alkyl oder Halogenalkyl mit jeweils 1 bis 6 Kohlenstoffatomen und gegebenenfalls 1 bis 9 gleichen oder verschiedenen Halogenatomen, insbesondere Fluor, Chlor, Brom steht, ferner für jeweils gegebenenfalls ein- bis dreifach, gleich oder verschieden im Cycloalkylteil durch Fluor, Chlor, Brom, Methyl oder Ethyl substituiertes Cyclopropyl, Cyclopropylmethyl, Cyclopropylethyl, Cyclopropylpropyl, Cyclobutyl, Cyclopentyl, Cyclohexyl oder Cyclohexylmethyl oder für jeweils gegebenenfalls ein- bis fünffach, gleich oder verschieden substituiertes Phenyl oder Naphthyl steht, wobei als Substituenten jeweils in Frage kommen: Fluor, Chlor, Brom, Cyano, Nitro, Methyl, Ethyl, n- oder i-Propyl, n-Butyl, Methoxy, Ethoxy, Chlormethyl, Dichlormethyl, Trichlormethyl, Trifluormethyl, Methoxymethyl, Methoxyethyl, Ethoxyethyl, Methoxypropyl, Ethoxypropyl, Methoxycarbonyl, Ethoxycarbonyl, Propoxycarbonyl, Butoxycarbonyl, Pentyloxycarbonyl, Methoxyethyloxycarbonyl oder Methoxymethoxycarbonyl.

Im einzelnen seien außer den bei den Herstellungsbeispielen genannten Verbindungen die folgenden N-(3-Chlor-1,2,4-oxadiazol-5-yl)-harnstoffe der allgemeinen Formel (I) genannt:

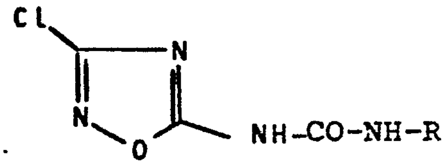 (I )

| R | R |
|---|---|
| $(CH_3)_2CH-CH_2-$ | 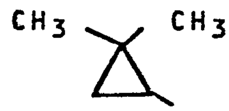 |
| $(CH_3)_2CH-CH_2-CH_2-$ | 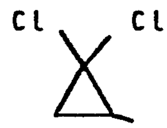 |
| $Cl-(CH_2)_6-$ | 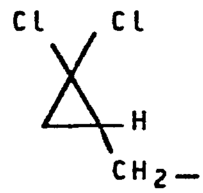 |
| 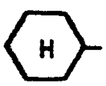 | 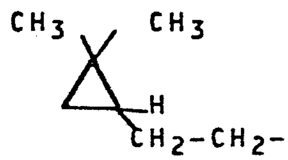 |
| 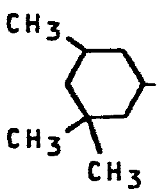 | |

| R | R |
|---|---|
| 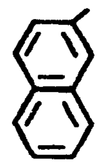 | 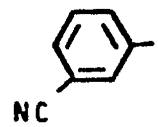 |
| 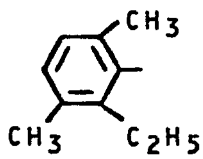 | 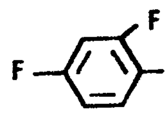 |
| 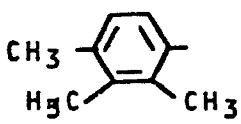 | 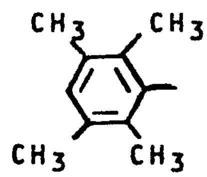 |
| 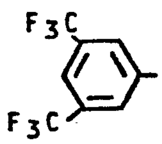 | 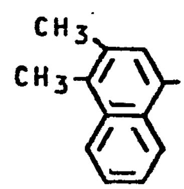 |
| 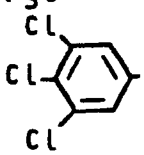 | 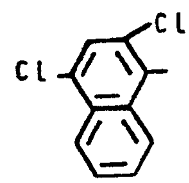 |
| 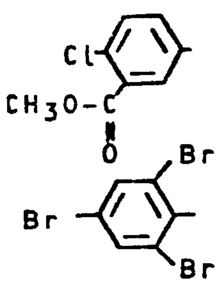 | 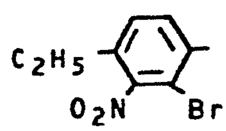 |
| 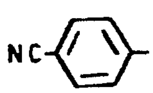 | |

Verwendet man beispielsweise 5-Amino-3-chlor-1,2,4-oxadiazol und Pentachlorphenylisocyanat als Ausgangsstoffe, so läßt sich der Reaktionsablauf des erfindungsgemäßen Verfahrens durch das folgende Formelschema darstellen:

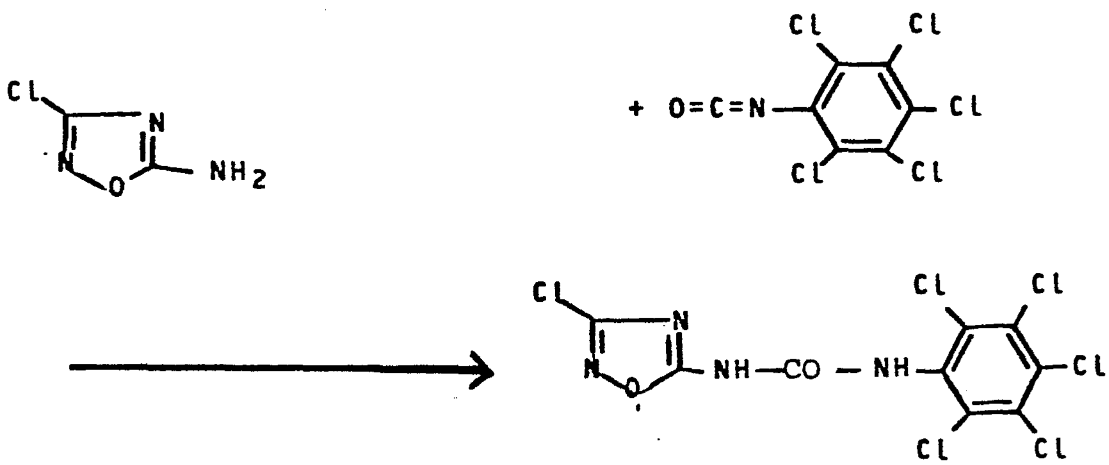

Das zur Durchführung des erfindungsgemäßen Verfahrens als Ausgangsverbindung benötigte 5-Amino-3-chlor-1,2,4-oxadiazol der Formel (II) ist noch nicht bekannt. Man erhält es, wenn man das bekannte 2,5-Dichlor-1,2,4-oxadiazol der Formel (IV),

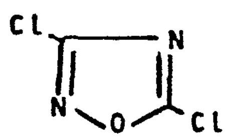

(IV)

mit wäßrigem Ammoniak bei Temperaturen zwischen -20°C und +30°C umsetzt.

Die zur Durchführung des erfindungsgemäßen Verfahrens weiterhin als Ausgangsverbindungen benötigten Isocyanate sind durch die Formel (III) allgemin definiert. In dieser Formel (III) steht R vorzugsweise für diejenigen Reste, die bereits im Zusammenhang mit der Beschreibung der erfindungsgemäßen Stoffe der Formel (I) für diesen Substituenten genannt wurden.

Die Isocyanate der Formel (III) sind allgemein bekannte Verbindungen der organischen Chemie oder lassen sich nach prinzipiell bekannten Verfahren in analoger Weise erhalten.

Als Verdünnungsmittel zur Durchführung des erfindungsgemäßen Verfahrens kommen inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethyleter, Dioxan, Tetrahydrofuran oder Ethylenglykoldimethyl- oder-diethylether; Ketone wie Aceton oder Butanon; Nitrile, wie Acetonitril oder Propionitril oder Amide, wie Dimethylformamid, Dimethylacetamid, N-Methylformanilid, N-Methylpyrrolidon oder Hexamethylphosphorsäuretriamid oder Dimethylsulfoxid.

Das erfindungsgemäße Verfahren kann gegebenenfalls in Gegenwart eines Katalysators durchgeführt werden.

Als solche kommen alle üblichen anorganischen oder organischen Basen in Frage. Hierzu gehören beispielsweise Alkalimetallcarbonate, wie Natriumcarbonat, Kaliumcarbonat oder Natriumhydrogencarbonat; sowie tertiäre Amine, wie Triethylamin, N,N-Dimethylanilin, N,N-Dimethylbenzylamin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen 20°C und 160°C, vorzugsweise zwischen 60°C und 140°C.

Zur Durchführung des erfindungsgemäßen Verfahrens setzt man pro Mol 5-Amino-3-chlor-1,2,4-oxadiazol der Formel (II) im allgemeinen 1 bis 3 Mol, vorzugsweise 1 bis 2 Mol an Isocyanat der Formel (III) und gegebenenfalls 0,001 bis 1,5 Mol an Katalysator ein. Die Reaktionsdurchführung, Aufarbeitung und Isolierung der Reaktionsprodukte der Formel (I) erfolgt nach allgemein üblichen Methoden.

Die erfindungsgemäßen Wirkstoffe weisen eine starke mikrobizide Wirkung auf und können zur Bekämpfung

von unerwünschten Mikroorganismen praktisch eingesetzt werden. Die Wirkstoffe sind für den Gebrauch als Pflanzenschutzmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft, aber nicht begrenzend, seien einige Erreger von pilzlichen Erkrankungen, die unter die oben aufgezählten Oberbegriffe fallen, genannt:

Botrytis-Arten, wie beispielsweise Botrytis cinerea;
Plasmopara-Arten, wie beispielsweise Plasmopara viticola;
Uromyces-Arten, wie beispielsweise Uromyces appendiculatus;
Sphaerotheca-Arten, wie beispielsweise Sphaerotheca fuliginea;
Venturia-Arten, wie beispielsweise Venturia inaequalis;
Podosphaera-Arten, wie beispielsweise Podosphaera leucotricha;
Phytophthora-Arten, wie beispielsweise Phytophthora infestans;
Erysiphe-Arten, wie beispielsweise Erysiphe graminis;
Puccinia-Arten, wie beispielsweise Puccinia recondita;
Fusarium-Arten, wie beispielsweise Fusarium culmorum;
Ustiliago-Arten, wie beispielsweise Utilago nuda oder Ustilago avenae;
Septoria-Arten, wie beispielsweise Septoria nodorum;
Tilletia-Arten, wie beispielsweise Tilletia caries;

Pyricularia-Arten, wie beispielsweise Pyricularia oryzae; Pellicularia-Arten, wie beispielsweise Pellicularia sasakii;
Erwinia-Arten, wie beispielsweise Erwinia amylovora;
Pyrenophora-Arten, wie beispielsweise Pyrenophora teres; (Konidienform; Drechslera, Syn: Helminthosporium);
Leptosphaeria-Arten, wie beispielsweise Leptosphaeria nodorum;
Cochliobolus-Arten, wie beispielsweise Cochliobolus sativus (Konidienform: Drechslera, Syn: Helminthosporium) und
Cercospora-Arten, wie beispielsweise Cercospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, beispielsweise verursacht durch Leptosphaeria nodorum, Cochliobolus sativus, Fusarium-Arten, Pyrenophora teres, zur Bekämpfung von Reiskrankheiten, wie beispielsweise gegen den Erreger der Reisfleckenkrankheit [Pyricularia oryzae], sowie zur Bekämpfung von Rebenkrankheiten, wie beispielsweise gegen den Erreger des falschen Rebenmehltaus (Plasmopara viticola) ein-

setzen. Dabei zeigen die erfindungsgemäßen Wirkstoffe neben hervorragenden protektiven Eigenschaften auch sehr gute systemische Wirksamkeit.

In entsprechend höheren Aufwandmengen zeigen die erfindungsgemäßen Wirkstoffe auch herbizide Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additve können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht

in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

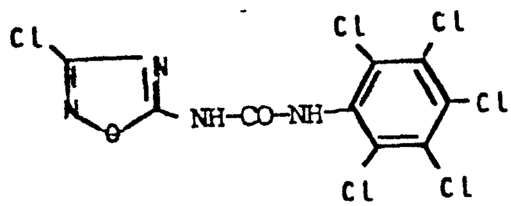

11,9 g (0,1 Mol) 5-Amino-3-chlor-1,2,4-oxadiazol und 29,1 g (0,1 Mol) Pentachlorphenylisocyanat werden zusammen mit wenigen Tropfen Dimethylbenzylamin in 100 ml trockenem Toluol 4 Stunden unter Rückfluß erhitzt. Nach dem Abkühlen auf Raumtemperatur saugt man den ausgefallenen Feststoff ab und wäscht mehrfach mit Ether nach.

Man erhält 32 g (78 % der Theorie) an N-(3-Chlor-1,2,4-oxadiazol-5-yl)-N'-pentachlorphenyl-harnstoff vom Schmelzpunkt 258°C.

In entsprechender Weise und gemäß den allgemeinen Angaben zur Herstellung erhält man die folgenden N-(3-Chlor-1,2,4-oxadiazol-5-yl)-harnstoffe der allgemeinen Formel (I):

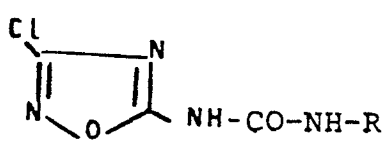

(I)

| Bsp. Nr. | R | Schmelzpunkt[°C] |
| --- | --- | --- |
| 2 | 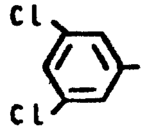 | 235 |
| 3 | 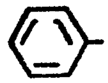 | 164 |
| 4 | 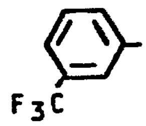 | 170-175 |
| 5 | 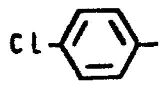 | 194-197 |
| 6 | 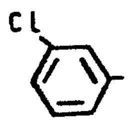 | 199-202 |
| 7 | 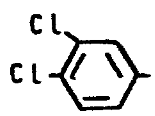 | 195-197 |
| 8 | 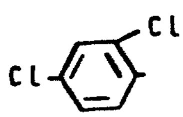 | 186-190 |
| 9 | 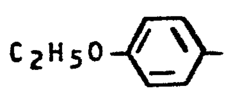 | 162-163 |
| 10 | 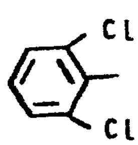 | 192 (Zers.) |
| 11 | 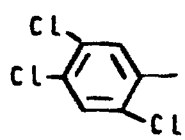 | 219-230 |

| Bsp. Nr. | R | Schmelzpunkt/°C |
| --- | --- | --- |
| 12 | 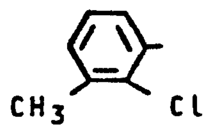 | 178-179 |
| 13 | 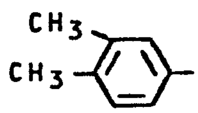 | 172-175(Zers.) |
| 14 | 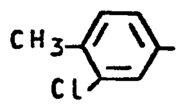 | 132-135 |
| 15 | 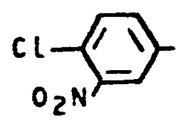 | > 186 (Zers.) |
| 16 | 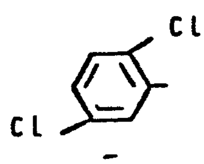 | > 250 (Zers.) |
| 17 | 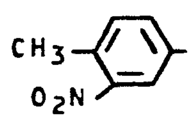 | 160 |
| 18 | 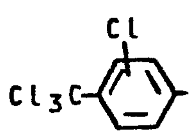 | 155-159 (Zers.) (einheitliche Substanz Zuordnung des Chloratoms nicht möglich) |
| 19 | 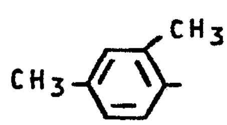 | 159 |
| 20 | 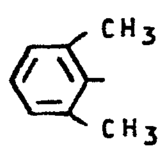 | 140-145 |
| 21 | 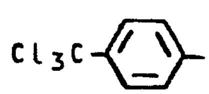 | > 250 |

| Bsp. Nr. | R | Schmelzpunkt/°C |
|---|---|---|
| 22 | 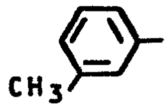 | > 176 (Zers.) |
| 23 | $CH_3-(CH_2)_2-$ | 147-148 |
| 24 | $CH_3$ | 208 |
| 25 | $C_2H_5$ | 173-176 |
| 26 | $CH_3-(CH_2)_3-$ | 140 |
| 27 | 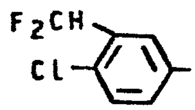 | 217-221 |
| 28 | 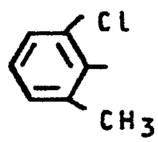 | > 247 (Zers.) |
| 29 | 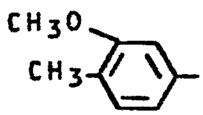 | 145-153 |
| 30 | 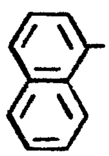 | > 248 (Zers.) |
| 31 | 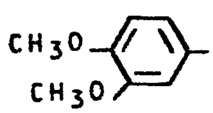 | > 218 (Zers.) |
| 32 | 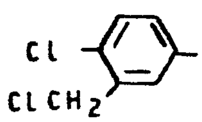 | > 186 (Zers.) |

| Bsp. Nr. | R | Schmelzpunkt/°C |
|---|---|---|
| 33 | 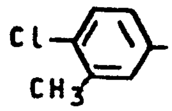 | > 186 (Zers.) |
| 34 | 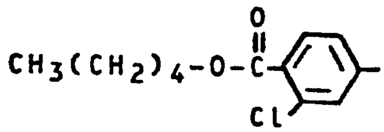 | Oel |
| 35 | 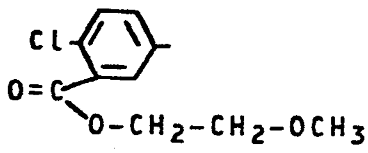 | 107-111 |
| 35 | 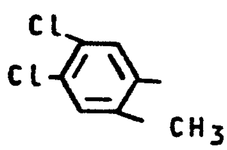 | 237 |
| 37 | 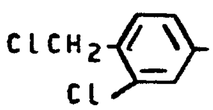 | > 138 (Zers.) |
| 38 | 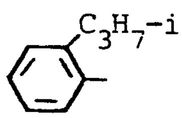 | > 162 (Zers.) |
| 39 | 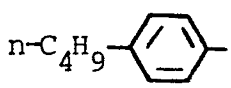 | 155-58 |
| 40 | 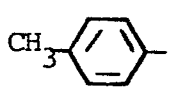 | > 194 (Zers.) |
| 41 | 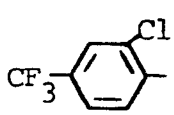 | 171-75 |

Herstellung der Ausgangsverbindung

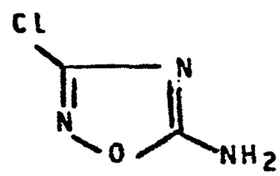

100 g (0,72 Mol) 3,5-Dichlor-1,2,4-oxadiazol werden tropfenweise unter Rühren bei 20°C (Kühlung) mit einer Mischung aus 108 g (0,8 Mol) 25prozentiger wäßriger Ammoniaklösung und 100 ml Wasser versetzt. Die Zugabe dauert ca. 1,5 Stunden. Nach beendeter Zugabe rührt man weitere 3 Stunden bei Raumtemperatur nach, saugt den ausgefallenen Niederschlag ab und kristallisiert aus Wasser um.

Man erhält 67,8 g (79 % der Theorie) an 5-Amino-3-chlor-1,2,4-oxadiazol vom Schmelzpunkt 169°C-171°C.

## Anwendungsbeispiele

In den folgenden Anwendungsbeispielen wurden die nachstehend aufgeführten Verbindungen als Vergleichssubstanzen eingesetzt:

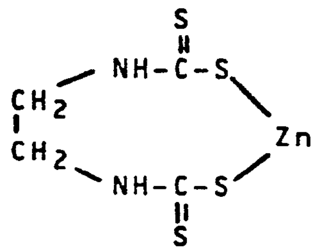

(A)

Zink-ethylen-1,2-bis-(dithiocarbamat) und

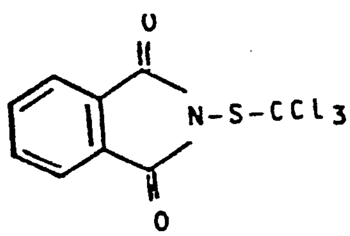

(B)

N-Trichlormethylthio-phthalimid

0185983

Beispiel A

Pyricularia-Test (Reis) / systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 1, 10, 17, 25.

0185983

Beispiel B

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 1, 2, 5, 7, 8, 11.

Beispiel C

Leptosphaeria nodorum-Test (Weizen) / protektiv

| | | | |
|---|---|---|---|
| Lösungsmittel: | 100 | Gewichtsteile | Dimethylformamid |
| Emulgator: | 0,25 | Gewichtsteile | Alkylarylpolyglykol-ether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden im Gewächshaus bei einer Temperatur von ca. 15°C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 2, 5, 7.

Beispiel D

Plasmopara-Test (Reben) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100 % relativer Luftfeuchtigkeit. Anschließend werden die Pflanzen 5 Tage im Gewächshaus bei 22°C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 2, 7, 8.

0185983

## Patentansprüche

1. N-(3-Chlor-1,2,4-oxadiazol-5-yl)-harnstoffe der allgemeinen Formel (I),

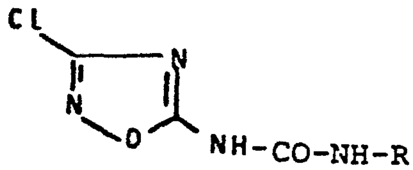

(I)

in welcher

R für Alkyl, Halogenalkyl, für jeweils gegebenenfalls substituiertes Cycloalkyl oder Cycloalkylalkyl oder für gegebenenfalls substituiertes Aryl steht.

2. N-(3-Chlor-1,2,4-oxadiazol-5-yl)-harnstoffe der Formel (I) gemäß Anspruch 1,

in welcher

R für jeweils geradkettiges oder verzweigtes Alkyl oder Halogenalkyl mit jeweils 1 bis 12 Kohlenstoffatomen und gegebenenfalls 1 bis 15 gleichen oder verschiedenen Halogenatomen, für jeweils gegebenenfalls einfach oder mehrfach, gleich oder verschieden im Cyloalkylteil durch Halogen und/oder geradkettiges oder verzweigtes Alkyl mit 1 bis 4 Kohlenstoffatomen substituiertes Cycloalkyl oder Cycloalkylalkyl mit jeweils 3 bis 7 Kohlenstoffatomen im Cycloalkylteil und gegebenenfalls 1 bis 6 Kohlenstoffatomen im Alkylteil oder für gegebenenfalls einfach oder mehrfach, gleich oder verschieden substituiertes Aryl mit 6 bis 10 Kohlenstoffatomen steht, wobei als Arylsubstituenten in Frage kommen: Halogen, Cyano, Nitro, sowie jeweils geradkettiges oder verzweigtes Alkyl, Alkoxy, Halogenalkyl, Alkoxyalkyl, Alkoxyalkyloxycarbonyl oder Alkoxycarbonyl mit jeweils 1 bis 8 Kohlenstoffatomen in den einzelnen Alkyl- bzw. Alkoxyteilen und gegebenenfalls 1 bis 9 gleichen oder verschiedenen Halogenatomen.

3. N-(3-Chlor-1,2,4-Oxadiazol-5-yl)-harnstoffe der Formel (I) gemäß Anspruch 1,

in welcher

R für jeweils geradkettiges oder verzweigtes Alkyl oder Halogenalkyl mit jeweils 1 bis 6 Kohlenstoffatomen und gegebenenfalls 1 bis 9 gleichen oder verschiedenen Halogenatomen steht, ferner für jeweils gegebenenfalls ein- bis dreifach, gleich oder verschieden im Cycloalkylteil durch Fluor, Chlor, Brom, Methyl oder Ethyl substituiertes Cyclopropyl, Cyclopropylmethyl, Cyclopropylethyl, Cyclopropylpropyl, Cyclobutyl, Cyclopentyl, Cyclohexyl oder Cyclohexylmethyl oder für jeweils gegebenenfalls ein- bis fünffach, gleich oder verschieden substituiertes Phenyl oder Naphthyl steht, wobei als Substituenten jeweils in Frage kommen: Fluor, Chlor, Brom, Cyano, Nitro, Methyl, Ethyl, n- oder i-Propyl, n-Butyl, Methoxy, Ethoxy, Chlormethyl, Dichlormethyl, Trichlormethyl, Trifluormethyl, Methoxymethyl, Methoxyethyl, Ethoxyethyl, Methoxypropyl, Ethoxypropyl, Methoxycarbonyl, Ethoxycarbonyl, Propoxycarbonyl, Butoxycarbonyl, Pentyloxycarbonyl, Methoxyethyloxycarbonyl oder Methoxymethoxycarbonyl.

4. Verfahren zur Herstellung von N-(3-Chlor-1,2,4-oxadiazol-5-yl)-harnstoffen der allgemeinen Formel (I),

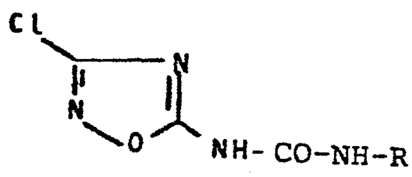

(I)

in welcher

R für Alkyl, Halogenalkyl, für jeweils gegebenenfalls substituiertes Cycloalkyl oder Cycloalkylalkyl oder für gegebenenfalls substituiertes Aryl steht,

dadurch gekennzeichnet, daß man 5-Amino-3-chlor-1,2,4-oxadiazol der Formel (II),

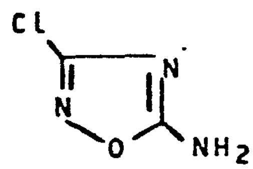

(II)

mit Isocyanaten der Formel (III),

R-N=C=O (III)

in welcher

R die oben angegebene Bedeutung hat,

gegebenenfalls in Gegenwart eines Verdünnungsmittels sowie gegebenenfalls in Gegenwart eines Katalysators umsetzt.

5. Schädlingsbekämpfungsmittel, gekennzeichnet durch einen Gehalt an mindestens einem N-(3-Chlor-1,2,4-Oxadiazol-5-yl)-harnstoff der Formel (I) gemäß den Ansprüchen 1 und 4.

6. Verfahren zur Bekämpfung von Schädlingen dadurch gekennzeichnet, daß man N-(3-Chlor-1,2,4-Oxadiazol-5-yl)-harnstoffe der Formel (I) gemäß den Ansprüchen 1 und 4 auf Schädlinge und/oder ihren Lebensraum einwirken läßt.

7. Verwendung von N-(3-Chlor-1,2,4-Oxadiazol-5-yl)-harnstoffen der Formel (I) gemäß den Ansprüchen 1 und 4 zur Bekämpfung von Schädlingen.

8. Verfahren zur Herstellung von Schädlingsbekämpfungsmitteln, dadurch gekennzeichnet, daß man N-(3-Chlor-1,2,4-Oxadiazol-5-yl)-harnstoffe der Formel (I) gemäß den Ansprüchen 1 und 4 mit Streckmitteln und/oder oberflächenaktiven Mitteln vermischt.

9. 5-Amino-3-chlor-1,2,4-Oxadiazol der Formel (II)

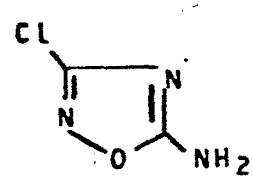

(II).

10. Verfahren zur Herstellung von 5-Amino-3-chlor-1,2,4-oxadiazol der Formel (II)

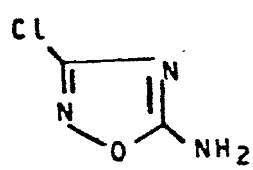

dadurch gekennzeichnet, daß man 2,5-Dichlor-1,2,4-oxadiazol der Formel (IV)

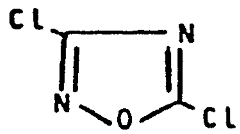

(IV)

mit wäßrigem Ammoniak bei Temperaturen zwischen -20°C und +30°C umsetzt.

Europäisches Patentamt

# EUROPÄISCHER RECHERCHENBERICHT

0185983
Nummer der Anmeldung

EP 85115352.8

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | DE - A1 - 2 801 509 (SCHERING)<br>* Anspruch 1 *<br>-- | 1 | C 07 D 271/06<br>A 01 N 47/28 |
| A | EP - A1 - 0 111 442 (CIBA-GEIGY)<br>* Verbindungen Nr. 2.1-2.8, 2.37-2.45 *<br>-- | 1 | |
| A | CH - A5 - 568 998 (BAYER)<br>* Anspruch; Spalte 3, Zeilen 31-38 *<br>---- | 1,5 | |
| | | | RECHERCHIERTE SACHGEBIETE (Int. Cl.4) |
| | | | C 07 D 271/00 |

Der vorliegende Recherchenbericht wurde für alle Patentansprüche erstellt

| Recherchenort | Abschlußdatum der Recherche | Prüfer |
|---|---|---|
| WIEN | 21-02-1986 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82